# EUROPEAN PATENT APPLICATION

(11) **EP 4 410 337 A2**
(43) Date of publication of application: **07.08.2024**
(21) Application number: 24170586.2
(22) Date of filing: 06.01.2021
(51) Int. Cl.: A61M 5/42

(54) **CANNULA INSERTION SYSTEM**

(30) Priority: 07.01.2020 NL 2024619
(62) Divisional of application: 21701219.4
(71) Applicant: Vitestro Holding B.V., 3526 KS Utrecht (NL)
(72) Inventor: OVERBEEKE, Toon Olaf, 3533 AB Utrecht (NL); JOSEPH, Brian Robert, 2597 EZ 'S Gravenhage (NL); KARSSEN, Johannes Gerhard Daniël, 3526 KS Utrecht (NL); JONKER, Arris Cornelis, 3526 KS Utrecht (NL); DE GREEF, Mark, 3526 KS Utrecht (NL); VAN SCHIE, Guido, 3526 KS Utrecht (NL); WOLKOTTE, Pascal Theodoor, 3526 KS Utrecht (NL); GIESEN, Luuk Frans Paul, 3526 KS Utrecht (NL); BEEKHUIS, Johan Houdijn, 3526 KS Utrecht (NL); DE BOER, Tomas, 3526 KS Utrecht (NL); BAX, Laurie Nesia Johanna, 3526 KS Utrecht (NL)
(74) Representative: EP&C

(57) **Abstract**

The present invention relates to a cannula insertion system to insert a cannula into a human or animal body, comprising:
one or more sensors to determine a suitable location for insertion of the cannula,
a cannula insertion device configured to insert the cannula into the human or animal body,
a cannula insertion device positioning system to support and position the cannula insertion device, and
a control device arranged to control the positioning system to position, on the basis of the determined location for insertion of the cannula, the cannula insertion device in a suitable position to insert the cannula into human or animal body.

## Description

The present invention relates to a cannula insertion system for inserting a cannula into a blood vessel of a human or animal.

US2008/0275396 A1 discloses an automated cannula insertion system for autonomously inserting a cannula into a blood vessel of a human or an animal, the contents of which are herein incorporated by reference in its entirety.

The cannula insertion system of US2008/0275396 A1 comprises an acquisition module that allows for determining at least a location of a blood vessel underneath the skin and is enabled to determine a puncture location that is suitable for inserting a cannula into the blood vessel. The cannula insertion system comprises an actuator for moving and aligning the cannula to a determined position and to autonomously insert the cannula into the blood vessel for multiple purposes, such as blood withdrawal, intravenous medication and infusions.

It is desirable that an automated cannula insertion system for autonomously inserting a cannula into a blood vessel of a human or an animal, is safely operated. In particular when a needle is inserted into a blood vessel, unnecessary damage to the blood vessel and the tissues surrounding the blood vessel should be avoided. Moreover, since the insertion of the cannula into the blood vessel may be performed completely automatically, an operator of the cannula insertion system may not or not timely react to any malfunctioning of the cannula insertion system which may introduce a practical risk to the use of an automated cannula insertion system.

It is an object of the invention to provide an improved cannula insertion system. In particular, it is an object of the invention to provide a cannula insertion system that allows to efficiently draw blood from a blood vessel of a human or animal body.

The invention provides a cannula insertion system to insert a cannula into a human or animal body, comprising:
one or more sensors to determine a suitable location for insertion of the cannula,
a cannula insertion device configured to insert the cannula into the human or animal body,
a cannula insertion device positioning system to support and position the cannula insertion device, and
a control device arranged to control the positioning system to position, on the basis of the determined location for insertion of the cannula, the cannula insertion device in a suitable position to insert the cannula into human or animal body.

In an embodiment, the cannula insertion system comprises a blood collection system to collect blood in one or more blood collection tubes supported by the blood collection system,
wherein the blood collection system is movably arranged to at least partly follow movements of the cannula insertion device.

In this embodiment of the cannula insertion system, the blood collection system is movable to follow at least a part of the movements of the cannula insertion device. This facilitates that the blood collection system will remain relatively close to the cannula insertion device during insertion of the cannula into a blood vessel of a human or animal body. This has the advantage that the distance that has to be bridged by blood drawn from the blood vessel to a blood collection tube can be held relatively small. This reduces the volume of blood that has to be used to fill a conduit between the patient and the blood collection tube, and as a result actual filling of blood collection tubes can more quickly be started after insertion of the cannula into the blood vessel. In addition, the drawing of blood is usually based on a vacuum provided in the blood collection tubes. When a relatively long conduit is used between patient and the blood collection tube, the vacuum of the first blood collection tube to be filled is to a large extent used for filling the conduit between the patient and the blood collection tube. This may also result in insufficient filling of this first blood collection tube with the result that the first blood collection tube cannot be reliably used for analysis of the blood.

The blood collection system may only follow larger movements of the cannula insertion device to keep the distance between the cannula insertion device and the blood collection system relatively small. Fine positioning movements of the cannula insertion device, such as for adjusting the insertion angle of the cannula, do not necessarily have to be followed by the blood collection system. The blood collection system may for example follow movements of the cannula insertion device in one or two main directions of movement.

In an embodiment, the blood collection system is mounted on the cannula insertion device positioning system to at least partly follow movements of the cannula insertion device. By mounting of the blood collection system on the cannula insertion device positioning system, no separate positioning system has to be provided to move the blood collection system in line with movements of the cannula insertion device. The cannula insertion device positioning system may be arranged to move the cannula insertion device in six degrees of freedom, while the blood collection system is mounted on the cannula insertion device positioning system at a location where it will follow movements in only one or two degrees of freedom, which are typically linear displacements. The blood collection system may have its own supporting system, i.e. the blood collection system may not be carried or fully carried by the cannula insertion device positioning system, but only have a mechanical link mounted to the cannula insertion device positioning system that pushes or pulls the blood collection system along with movements of the cannula insertion device positioning system. Such own supporting system may for example comprise guiding rails on which the blood collection system is at least partially supported.

In an alternative embodiment, the blood collection system may have its own positioning system which is arranged to move the blood collection system to follow movements of the cannula insertion device.

In an embodiment, the cannula insertion device positioning system is arranged to move the cannula insertion device between an operating position from which a cannula may be inserted into the human or animal body, and a retracted position. In some embodiments of a cannula insertion system, the cannula insertion device is only positioned in a position to insert a cannula into the human or animal body at a time interval where insertion of the cannula is desired. In the time between two patients being treated in the cannula insertion system, it may be advantageous to move the cannula insertion device from an operating position from which a cannula may be inserted into the human or animal body, to a retracted position. In this retracted position, the cannula is moved further away from a patient resulting in less risk that the patient is inadvertently touched by the cannula. Moreover, in the retracted position, the cannula insertion system may be arranged to exchange disposable parts of the cannula insertion system, such as the cannula and the blood collection tubes. In this retracted position, there will be typically more space available for exchange of disposable parts.

In an embodiment, the cannula insertion system may comprise a housing comprising a recess in which the cannula insertion device may be placed. During the insertion of the cannula into a patient, the cannula insertion device positioning system may be arranged in the operating position in which the cannula insertion device extends at least partially outside the housing. After blood withdrawal from the patient is finished, the cannula insertion device may be moved to the retracted position so that the cannula insertion device no longer extends from the housing. In the retracted position, within the housing, the cannula insertion system may exchange disposable parts such as cannula and blood collection tubes filled during blood withdrawal. Also, new disposable parts may be provided to facilitate blood withdrawal of a subsequent patient.

In alternative embodiments, the cannula insertion device may extend from the housing in both the operating position and the retracted position, whereby the cannula insertion device will further project from the housing in the operating position. Also, it is possible that the cannula insertion device is arranged in the housing in both the operating position and the retracted position, but that the cannula insertion device is more easily accessible from the outside of the housing, for instance with respect to an arm inserted into the housing.

In an embodiment, the cannula insertion device positioning system comprises at least one movable stage, for example a linear stage, wherein the blood collection system is mounted on the at least one movable stage. The stage is a part of the cannula insertion device positioning system that is movable by one or more actuators in one or more directions. By mounting the blood collection system on this stage, the blood collection system may follow movements of the stage, and therewith movements of the cannula insertion device. The stage is advantageously used for larger movements of the cannula insertion device so that the blood collection system will follow these larger movements of the cannula insertion device. The stage may for example be used to move the cannula insertion device between an operating position and a retracted position of the cannula insertion device. As a result, the blood collection system will move together with the cannula insertion device between the operating position and the retracted position.

In an embodiment, the cannula insertion device positioning system comprises three linear stages that are movable in three orthogonal directions and three rotation actuators to provide rotation about three rotation axes to allow movement of the cannula insertion device in six degrees of freedom.

In an embodiment, the cannula insertion system comprises a blood collection tube exchange system to exchange blood collection tubes between a blood collection tube input device and the blood collection system. During blood drawing of blood from a patient, it may be desirable that multiple blood collection tubes are filled. To handle these multiple blood collection tubes, i.e. supplying the tubes, loading and unloading the tubes in the blood collection system, the blood collection tube exchange system may be provided. The blood collection tube input device is a device that allows to introduce blood collection tubes into the cannula insertion system.

The blood collection tube input device is for example a blood collection tube storage and the blood collection tube exchange system may be configured to select the blood collection tubes from the blood collection tube storage in which multiple types of blood collection tubes are stored. In addition, or as an alternative, the blood collection tube exchange system may be configured to receive blood collection tubes that are manually inserted into the blood collection tube input device. These blood collection tubes may be inserted one-by-one into the blood collection tube input device, or as a set of pre-selected blood collection tubes. The set of pre-selected blood collection tubes may be selected for a specific set of blood test and/or a specific patient.

The set of pre-selected blood collections tubes may be arranged in a cartridge configured to hold one or more pre-selected blood collection tubes. The cartridge may define one or more holding positions for holding the one or more pre-selected blood collection tubes of the set of pre-selected blood collections tubes. The provision of a cartridge has the additional advantage that the blood collection tubes that are inserted in the blood collection tube input device will have a predetermined order. When drawing blood this order may be of relevance, for example to prevent contamination. It is therefore required to keep to this pre-determined order. By providing the cartridge with the set of pre-selected blood collection tubes, the correct sequence of the blood collection tubes will be followed.

The cartridge may be designed in such a way that the one or more blood collection tubes can be taken out of the cartridge when using a blood collection tube removal device or a cartridge opening device. This may prevent that a person may advertently or inadvertently take one or more blood collection tubes out of the cartridge.

The cartridge may for example be designed to allow easy manual placement of the blood collection tubes into the cartridge, but to prevent easy manual removal of the blood collection tubes out of the cartridge. The cannula insertion system may comprise a blood collection tube removal device to remove the blood collection tubes out of the cartridge. The cartridge opening device is for example a key or other element that is inserted in the cartridge for opening of the cartridge.

The advantage of the cartridge is that the set of pre-selected blood collections tubes will automatically be positioned in the correct position when the cartridge is placed in the blood collection tube input device. The cartridge may for example be filled for a specific patient at another location and be provided to a patient or physician. The patient or physician may carry the cartridge to the cannula insertion system and introduce the cartridge into the blood collection tube input device.

The blood collection tube exchange system may also be arranged to exchange blood collection tubes with a blood collection tube release device, where a blood collection tube or a set of pre-selected blood collections tubes, for example arranged in a cartridge can be taken out of the cannula insertion system. In practice, the blood collection tube input device and the blood collection tube release device may be integrated in a single device.

The blood collection system may further be configured to couple a selected blood collection tubes to the cannula to fill the blood collection tube to a desired filling level. After filling of the blood collection tube, the blood collection tube may be decoupled from the cannula and transported to a blood collection tube release device, where the blood collection tube can be taken out of the cannula insertion system.

In an embodiment, the blood collection system comprises an actuator to invert and/or rotate a blood collection tube during or after being filled with blood from the human or animal body.

In an embodiment, the blood collection system comprises a sensor arranged to measure a filling signal on the basis of which it can be determined whether the blood collection tube is sufficiently filled. This sensor is for example a level sensor that can measure whether a predetermined level of blood in the blood collection tube has been reached, for example by using a light beam. The sensor may also be configured to measure an actual filling level in the blood sensor, for example by measuring the weight of the blood collection tube.

When the blood collection tube is sufficiently filled, the blood collection tube may be decoupled from the cannula and, when desired, a new blood collection tube may be coupled to the cannula.

In an embodiment, the cannula insertion system comprises a blood collection tube reader device arranged to read information of the blood collection tube. The reader device may for example be a label reader that is capable of reading a label on the blood collection tube. It is also possible that the reader device is capable of reading a chip, such as a rfid chip, mounted on or in the blood collection tube. The reader device may also be a device capable of identifying one or more colors or patterns that are used to distinguish different types of blood collection tubes. For example, different types of blood collection tubes may be provided with caps of different colors so that the reader device may identify the type of blood collection tube by determining the color of the cap of the blood collection tube.

In an embodiment, the cannula insertion system comprises a blood collection tube labeling device. The blood collection tube labeling device may provide a label with relevant information on a blood collection tube or may print the relevant information on a label already present on the blood collection tube. The label may be provided or printed when the blood collection tube is just inserted into the cannula insertion system, or before, during or after the blood collection tube is being filled with blood.

The blood collection tube reader device and/or the blood collection tube labeling device may be part of the blood collection tube exchange system, the blood collection system, or provided at any other suitable location in the cannula insertion system.

In an embodiment, each cannula comprises a patient needle to be inserted into a patient, a tube needle to be inserted in a blood collection tube and a conduit connecting the patient needle and the tube needle. This cannula can be used to fill one or more blood collection tubes by inserting the blood collection tube in the one or more blood collection tubes.

The blood collection system may comprise a tube needle insertion device to insert the tube needle in the respective blood collection tube and pull the tube needle out of the blood collection tube after the blood collection tube has been filled with blood. The blood collection system may further comprise one or more holding locations to support the one or more blood collection tubes. The holding locations may be arranged in one or more rows that are movable by an actuator with respect to the needle insertion device to align a respective holding location of the holding locations with the needle insertion device to insert the tube needle in the respective holding location.

In an embodiment, the holding locations are arranged in a carousel that can be rotated with respect to the needle insertion device. A rotation actuator may be provided to rotate the carousel to align a selected holding location with the tube needle insertion device.

In an embodiment, the blood collection tube exchange system may, in the retracted position, exchange one or more blood collection tubes supported by the blood collection system.

In an embodiment, the system comprises a cannula exchange system to exchange a cannula held by the cannula insertion device, wherein the cannula exchange system is arranged to dispose a used cannula in a cannula disposal container. For each insertion of a cannula into a human or animal body a new disposable cannula should be used. It is advantageous that the cannula insertion system comprises a cannula exchange system that is arranged to automatically exchange a cannula that is held by the cannula insertion device.

The cannula exchange system may comprise a displacement device, for example a robotic arm, to move the cannula from an insertion area where the cannula is inserted into the human or animal body, to a discharge area where the cannula may be discharged into the cannula disposal container. The displacement device may be a separate device or may be the cannula insertion device positioning system. In an alternative embodiment, the cannula disposal container may be displaceable to a rest position and a cannula receipt position.

The cannula disposal container may be any container suitable to receive cannulas after their use. The cannula disposal container may be disposable itself, so that it can be disposed together with the used cannulas, or the cannula disposal container may be emptied and placed back into the cannula insertion system, preferably after cleaning and disinfection.

In an embodiment, the cannula exchange system is arranged to select the cannula from a cannula supply comprising multiple cannulas. The cannula exchange system may take for each insertion procedure a cannula from the cannula supply, and discharge it after use.

The displacement device of the cannula exchange system may be configured to displace a cannula between a supply area at or near the cannula supply where a cannula is taken from the cannula supply of multiple cannulas, to a loading area where the cannula is coupled to the cannula insertion device, and, potentially, a discharge area where the cannula may be discharged into the cannula disposal container.

The cannula supply may comprise multiple types of cannulas, for example cannulas having different needle diameters or lengths. The cannula exchange system may be configured to select a cannula of a desired type. This selection may for example depend on the basis of an identity of a person in which the cannula will be inserted.

In an alternative embodiment, the cannula may be manually placed in the cannula insertion device.

In an embodiment, the cannula exchange system may, in the retracted position, exchange a cannula with the cannula insertion device.

In an embodiment, the suitable location for insertion of a cannula is located on or in an arm of a human, and wherein the one or more sensors are arranged to determine a shape of the arm and/or a location of an elbow pit of the human. By determination of the shape of the arm and/or the location of an elbow pit of the human, the selection of a suitable insertion location may be facilitated. For example, the selection of a suitable insertion location for the cannula may be limited to an area at or close to the elbow pit of the human.

The one or more sensors arranged to determine a shape of the arm and/or a location of an elbow pit of the human, may for example comprise cameras or a contact sensor.

The images obtained by the cameras may be used to determine the shape of the arm and/or a location of an elbow pit by image recognition of the images.

The sensor signal obtained with a contact sensor, for example an ultrasound transducer, may also be used to determine the position of the contact sensor with respect to a skin surface. If no image of tissue is obtained with a part of the measurement surface of the contact sensor, this means that this part of the measurement surface is not in contact with tissue. By combining the images obtained with the contact sensor in multiple positions of the contact surface at least partly in contact with the skin surface, the shape of the arm and/or the location of an elbow pit can be determined.

In addition, or as an alternative, the contact sensor, for example an ultrasound transducer, may comprise sensors such as force sensors and/or distance sensors with which the position and/or shape of an arm surface with respect to the contact sensor may be measured.

In an embodiment, the cannula insertion system comprises an end-effector supporting at least one of the one or more sensors and the cannula insertion device. It may be advantageous to create a fixed spatial relationship between the cannula insertion device and the at least one of the one or more sensors that are used to determine a suitable location for introduction of a cannula into the human or animal body. The at least one sensor may be a contact sensor, for example an ultrasound transducer.

The end-effector may be mounted on the cannula insertion device positioning system, i.e. the cannula insertion device positioning system is used to position both the at least one sensor and the cannula insertion device. The cannula insertion device may comprise one or more cannula actuators to move the cannula with respect to the end-effector. The one or more cannula actuators may for example comprise a rotation actuator to adjust an insertion angle of the cannula and a linear actuator to translate the cannula along an insertion path.

In an embodiment, the cannula insertion system is arranged to insert a cannula into a vein of the human or animal body. The cannula insertion system may in particular be used to introduce autonomously or at least to a high degree automatically the cannula into a vein of a human or animal body. The cannula may for example be introduced for drawing blood of the patient, or for placement of an intravenous catheter or line into the vein of the human or animal body.

In an embodiment, the one or more sensors provide at least one sensor signal, wherein the control device is arranged to create on the basis of the at least one sensor signal an image of a vein suitable for insertion of the cannula. To determine a suitable insertion path for a cannula, the one or more sensors may be used to provide a sensor signal with which an image may be determined. This image of the vein may be a 3-dimensional image or a series of two-dimensional images taken along the longitudinal extent of the vein.

In an embodiment, the cannula insertion system comprises a pressure applicator arranged to apply pressure on the penetration location after retraction of the cannula from the insertion location. After retraction of the cannula of the human or animal body, it may be needed to apply pressure on the penetration location where the cannula has penetrated the skin to stop bleeding caused by the introduction of the cannula into the human or animal body. The pressure may be applied by any device suitable to apply a pressure on the penetration location. The pressure applicator may comprise a pressure element, such as a pressure plate or pressure body which is pressed on the skin at the penetration location. The pressure applicator is advantageously configured to automatically place the pressure element at the penetration location and exert a suitable force with this pressure element.

The pressure applicator may have its own positioning system. In an alternative embodiment, the pressure applicator may be arranged on an end-effector to support the cannula insertion device and the at least one sensor, whereby positioning of the pressure element is carried out by the cannula insertion device positioning system. In addition, a separate actuator may be provided to move the pressure applicator with respect to the end-effector.

In an embodiment, the pressure applicator comprises a bandage support to support a bandage with which pressure is exerted on the insertion location. In this embodiment the pressure element may press a bandage which is arranged between the pressure element and the penetration location against the skin at the penetration location to stop bleeding of the patient. The use of a bandage has the advantage that the pressure element may not have to be cleaned each time when the pressure element is used to exert a pressure on the skin. Furthermore, the bandage support may be configured to hold the bandage until the pressure applicator is stopped to apply the pressure on the skin and then release the bandage. The bandage may remain on the skin of the human or animal body to be used to further stop bleeding.

The pressure applicator may comprise a tape dispenser to tape the bandage on the skin of the human or animal body. In an alternative embodiment the tape may be part of the bandage that is released by the pressure applicator. A bandage storage may be provided to store bandages. The bandage storage may be arranged to provide the bandage when desired.

In an embodiment, the system comprises a patient or treatment identification device. It may be advantageous that the cannula insertion system can be fed with information of a patient to be treated by the cannula insertion system or with information of a treatment to be given. To feed this information into the system, the system may comprise a patient or treatment identification device to identify a patient to be treated or a treatment to be given to a patient. This patient or treatment identification device may for example comprise a reader or scanner that can read/scan relevant information such as an ID, passport, hospital ID, hospital letter or form with barcode, insurance card, etc.

The patient or treatment identification device may be connected to electronic patient record and /or a laboratory information system and may receive all data from these systems required for that patient for treatment in the cannula insertion system.

The patient or treatment identification device may also be configured to only be informed with respect to the treatment to be given to the human or animal body without the need of a personal identification of the patient.

On the basis of the identification of the patient or treatment, the cannula insertion system may be configured to carry out different actions specific for this patient and/or treatment, such as selection of a cannula type, selection of an insertion location, selection of blood collection tubes, etc. The information may also be used to label the blood collection tubes with personal information.

In an embodiment, the cannula insertion system comprises a hand grip to be held by a hand of a patient, wherein the hand grip may be configured to measure a holding force with which the hand grip is held by the patient and/or a holding position in which the hand grip is held by the patient.

It may be of importance to monitor the patient's well-being during the insertion of a cannula into the patient. It may be possible that a patient faints during the cannula insertion procedure. In such case it is undesirable that the procedure continues. To determine the well-being of a patient, a hand grip may be provided which is held by the patient during the insertion of the cannula into the patient, for example an arm of a patient.

The hand grip, or at least a part thereof, may be movable between a stand-by position and an activated position, for example by a sliding movement between the stand-by position and the activated position. The hand grip or the movable part thereof may be biased to the stand-by position. To allow the procedure to start and proceed, the patient must hold the hand grip in the activated position. If the patient faints the holding position of the hand grip may change from the activated position to the stand-by position. The cannula insertion system may monitor during the procedure the holding position of the hand grip or the movable part thereof, and abort the procedure when the hand grip or the movable part thereof, is moved from the activated position to the stand-by position.

In an alternative embodiment, the holding force with which the hand grip is held during the procedure may be monitored and be used as an indicator for the well-being of the patient.

In another alternative embodiment, one or more cameras may be used to monitor the position of the arm or other body parts of the patient. The cannula insertion system may be arranged to abort the procedure in case, on the basis of the images of the one or more cameras, it is determined that the patients faints or makes other movements, such as shaking movements, that may trigger a decision to abort the procedure.

In an embodiment, the cannula insertion system is arranged to carry out a cannula insertion procedure if the holding force is within a predefined holding force range, and to abort the cannula insertion procedure if the holding force is outside the predefined holding force range. For example, the cannula insertion system may be arranged to carry out a cannula insertion procedure if the holding force is within a predefined holding force range having a lower threshold value and an upper threshold value, and to abort the cannula insertion procedure if the holding force is below the lower threshold value or above the upper threshold value.

In an embodiment, the cannula insertion system comprises a tourniquet device, wherein the tourniquet device comprises a safety release device to release the tourniquet from a patient's arm or to release the tourniquet from the cannula insertion system if a pulling force exceeds a pulling force threshold.

A tourniquet device comprises a band, strap or such which is placed around a limb, for example an arm of a patient, at a location proximal of the insertion location. The band or strap is tightened to apply a pressure on the limb such that blood will more easily enter the limb than leave the limb. The pressure may for example be applied by pulling the strap or band or by inflation of an inflatable band. As a result, the pressure in the veins and therewith volume of the veins will increase. This substantially improves the recognition of veins suitable for insertion of a cannula.

The tourniquet device may be arranged to automatically arrange the band or strap around the limb. In alternative embodiments, the strap or band has to be applied manually around the limb.

The tourniquet device may also be arranged to automatically adjust the pressure applied by the strap or band on the limb, for example by adjusting the pulling force on the strap or band, or by inflation/deflation or an inflatable band.

In an embodiment, the blood collection tube exchange system or blood collection system comprises a blood quantity sensor arranged to provide a blood quantity signal representative for a quantity of blood present in a blood collection tube. The blood quantity signal is for example a blood level measured with an optical sensor, or a weight sensor with which the weight of the blood collection tube can be measured.

In an embodiment, the one or more sensors are arranged to determine one or more locations of nerves, and wherein the control device is arranged to prevent the insertion of the cannula into the nerves. It is undesirable that a cannula being inserted into the human or animal body will contact a nerve. Therefore, it is advantageous that the location of nerves is determined before the insertion of the cannula into the human or animal body. The location of nerves can be taken into account when determining a cannula insertion path.

In an embodiment, the cannula insertion system comprises a blood presence sensor located near, on or in the cannula. This blood presence sensor is arranged to determine whether blood enters the cannula. This can be used to determine the entrance of the cannula into the blood vessel. This can be compared with the expected entrance of a blood vessel on the basis of the cannula insertion path as determined by the control device of the cannula insertion system. This confirmation of the entrance of the blood vessel can be used to determine whether the cannula insertion path is correctly followed. When there is a large difference between the actual entrance of a blood vessel as measured with a blood contact sensor and the expected entrance of a blood vessel on the basis of the cannula insertion path, the cannula insertion procedure may be stopped as a safety measure.

The blood presence sensor may be a small sensor near the cannula or a sensor which can measure through the cannula, for example by light detection through the needle or by capacity/electrical signals.

In an embodiment, the cannula insertion system may also be arranged to verify whether a detected blood vessel is a vein and not an artery. This can for example be done by using Doppler techniques or using variation of the force with which the contact sensor is pressed on the arm of a patient. Since veins will more easily compress than arteries, a vein can be distinguished from an artery.

Also, image recognition software could be used to determine flow characteristics of an artery and a vein, in particular the pulsation of flow in the detected blood vessels. In particular, when an upper arm is held in a tourniquet, a vein in the under arm will have no pulsating flow while the pulsating flow within an artery can be determined.

In an embodiment, the cannula insertion device may be provided with a force sensor with which the axial force exerted on the cannula may be measured. By measuring the axial force exerted on the cannula, it can be determined when the wall of a vein is penetrated by the cannula. Further, this axial force on the cannula may be compared with a maximum allowable axial force. Insertion of the cannula may be stopped or at least the insertion speed may be lowered when this axial force exceeds this maximum allowable axial force.

In an embodiment, the cannula comprises a patient interface to communicate with a patient and/or an operator interface to communicate with an operator.

In an embodiment, the cannula insertion system, or at least a part thereof, such as the end-effector and/or the contact sensor, may be placed in a cover to protect the cannula insertion system or the respective part thereof against blood and bodily fluids. The cover could be a plastic cover, a bandage or a special fabricated shield. The cannula insertion system may comprises an automatic cover changing device which is arranged to automatically arrange the cover on the cannula insertion system, or the respective part thereof, and to dispose it when desired.

In an embodiment, the cannula insertion system comprises an automatic cleaning device arranged to automatically clean the cannula insertion system, or at least a part thereof. The automatic cleaning device can clean and disinfect the cannula insertion system, in particular the parts touching the patient at the venipuncture location, such as contact sensors. This cleaning can be done by automated brushing, washing, or wiping of (part of) the end-effector. After cleaning, the respective parts can be dried to prepare for disinfection, for example through blowing of air or heating. Disinfection may occur through closed UV-C chamber for a predetermined amount of time; alternatively, disinfection occurs through automated spraying, wiping and/or soaking.

Sensors, for example one or more cameras can be arranged to check whether cleaning and disinfection is desired before the procedure starts and/or after the procedure.

The automatic cannula insertion system may also be configured as a biopsy device, a surgery robot, a laparoscopic robot, or any other system that may use an autonomous cannula insertion system in a body part of human or animal.

Further characteristics and advantages of the cannula insertion system of the invention will now be explained by description of an embodiment of the invention, whereby reference is made to the appended drawings, in which:
Figure 1 shows schematically a first part of a cannula insertion system according to an embodiment of the invention comprising an end-effector supporting a contact sensor and a cannula insertion device;
Figure 2 shows schematically a second part of the cannula insertion system according to an embodiment of the invention comprising a pressure applicator and arm rest;
Figure 3 shows schematically a third part of the cannula insertion system according to an embodiment of the invention comprising a cannula exchange system;
Figure 4 shows schematically a fourth part of the cannula insertion system according to an embodiment of the invention comprising a blood collection tube exchange system; and
Figure 5 and 6 show an embodiment of a cannula insertion system according to an embodiment of the invention; and
Figure 7 shows an embodiment of a blood collection system according to an embodiment of the invention.

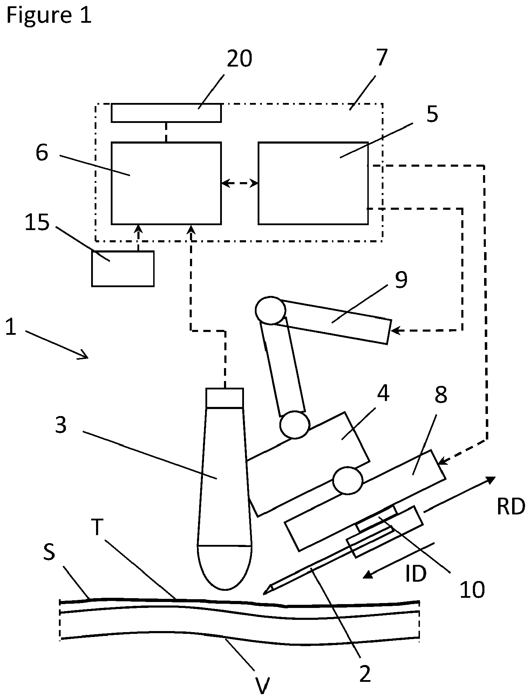

Figures 1-4 show parts of a cannula insertion system, generally denoted by reference numeral 1. Figures 1-4 are used to show different parts of a cannula insertion system 1 to describe/show different elements/devices of the cannula insertion system 1 and explain their function. In practice, the different devices/element may be arranged close to each other and would be visible in the different Figures. Thus, some parts shown in one or more of the figures are deliberately not shown in other Figures. Also, not all devices/elements are drawn to scale with respect to the other devices/elements shown in the same or other Figures.

The cannula insertion system 1 is configured to autonomously insert a cannula 2, for example a needle into a blood vessel V of a human or animal. The cannula insertion system 1 as shown in Figure 1 is in particular configured to autonomously draw blood from a blood vessel. In alternative embodiments, the cannula insertion system 1 may be configured to arrange a cannula in a blood vessel V for intravenous medication and/or infusions.

To draw blood from the blood vessel V, the cannula insertion system 1 may be arranged to determine a location of a blood vessel underneath the skin S of the human or animal, insert a cannula 2 into the blood vessel V and draw blood from the blood vessel V without direct interaction of an operator of the cannula insertion system 1.

The cannula insertion system 1, shown on Figure 1, comprises an ultrasound transducer 3 to obtain one or more sensor signals that are representative for the location of a blood vessel V in the human or animal. The ultrasound transducer 3 is a contact sensor. During use contact between the ultrasound transducer 3 and the skin S is required in order to obtain relevant data with respect to the location of the blood vessel V. The ultrasound transducer may be guided along the skin S of the human or animal in a target area T. This target area T is an area of the skin S underneath which the presence of a blood vessel V suitable for insertion of a cannula 2 is expected and which is examined by the ultrasound transducer 3. A control device 5 controls the position of the ultrasound transducer 3.

The target area T may be determined by obtaining images of the skin of a human or animal, for example using NIR (near infrared), infrared or visible light sensors 15, and determining on the basis of the images an area in which it is likely that a blood vessel suitable for insertion of a cannula will be found. The cannula insertion system 1 has multiple fixed sensors, i.e. sensors mounted at a fixed location, such as sensor 15. These multiple fixed sensors may be used to determine the target area, i.e. an approximate location of a blood vessel that can be used for insertion of the cannula 2. In an alternative embodiment, one or more of these NIR or visible light sensors may be arranged on a movable end-effector 4.

In addition, or as an alternative the fixed sensors 15 may be used to determine a location of the elbow pit of the arm and/or the shape of the arm. The sensors 15 could also be used to detect areas which should not be used for venipuncture, like birthmarks and wounds.

Once a target area T is determined, the ultrasound transducer 3 may be used to provide a sensor signal representative for a location of the blood vessel V within the target area T. The sensor signal is fed into a processing device 6 which is arranged to process the sensor signal. The control device 5 and the processing device 6 may be comprised in a single processor 7, such as a PC.

On the basis of the sensor signal provided by the ultrasound transducer 3, an image of the vein may be created by the processing device 6. This image may be based on multiple 2D images along a line in a certain direction, 3D coordinates and direction, 6D coordinates or may be a 3D image.

The processing device 6 determines on the basis of the sensor signal, or sensor signals, the location of a blood vessel V suitable for the insertion of the cannula 2. On the basis of this location, the processing device 6 may determine an insertion path for insertion of the cannula 2 into the blood vessel V. The processing device 6 may also locate nerves and prevent from penetrating those nerves, when selecting an insertion path for the cannula 2.

If no suitable location for insertion of the cannula 2 can be found, the cannula insertion system 1 may request the patient to place the other arm in the cannula insertion system 1.

The cannula 2 is arranged on a cannula insertion device 8. The cannula 2 is held by a cannula holder 10. The cannula 2 can be taken out of the cannula holder to facilitate exchange of cannulas 2.

The cannula insertion device 8 is arranged to insert the cannula 2 in an insertion direction ID through the skin S and into the blood vessel V along the insertion path determined by the processing device 6. The cannula insertion device comprises a rotation actuator to adjust an insertion angle of the cannula 2 to align a longitudinal axis of the cannula 2 with the insertion direction ID. A linear actuator is provided to translate the cannula 2 along an insertion path in the insertion direction ID.

The insertion path of the cannula 2 may be adapted to anatomical structures in the arm of the patient. By adapting the position of the cannula insertion device 8 during insertion of the cannula 2, the cannula 2 may also be moved along a non-linear insertion path.

The cannula insertion device 8 may be provided with a force sensor with which the axial force exerted on the cannula 2 may be measured. By measuring the axial force exerted on the cannula 2, it can be determined when the wall of a vein is penetrated by the cannula 2. Further, this axial force on the cannula 2 may be compared with a maximum allowable axial force. Insertion of the cannula 2 may be stopped or at least the insertion speed may be lowered when this axial force exceeds this maximum allowable axial force.

The cannula insertion device 8 may comprise a safety device arranged to retract the cannula 2 or to release the cannula 2 from the cannula insertion device 8 when a force exerted on the cannula 2 in a direction perpendicular to the insertion direction ID results in exceeding a respective safety threshold value. The release of the cannula 2 from the cannula insertion device 8 may be a complete release or a release in a limited number of degrees of freedom, for example one or two rotation directions. The safety device may for example allow to freely rotate in the rotation direction in which a torque is exerted on the cannula when this torque exceeds a safety threshold value in order to follow an inadvertent movement of the human or animal body.

The cannula insertion device 8 is supported by a positioning system 9, for example a robot arm, that is arranged to bring the cannula insertion device 8 in a position from which the cannula insertion device 8 may move the cannula 2 along the insertion path. The cannula insertion device 8 and the positioning system 9 are controlled by the control device 5.

In some cases, a vein positioned below the ultrasound transducer 3 may displace due to the pressure exerted with the ultrasound transducer 3. This may also be referred to as a 'rolling vein'. When this occurs, this may be determined on the basis of the measurements of the ultrasound transducer 3. The cannula insertion system 1 may be configured to control the cannula insertion device 8 to actively follow the movement of the vein. In addition, or as an alternative, a mechanism may be provided to prevent displacement of the vein, for example by arranging two mechanical elements on the skin surface at opposite sides of the vein. Also, it is possible to abort the cannula insertion procedure when displacement of the vein is measured by the ultrasound transducer 3.

In the embodiment shown in Figure 1, the ultrasound transducer 3 and the cannula insertion device 8 are mounted on a single end-effector 4. This ensures a fixed spatial relationship between the ultrasound transducer 3 and the cannula insertion device 8. The positioning system 9 is therefore used for positioning of both the ultrasound transducer 3 and the cannula insertion device 8. In an alternative embodiment, the ultrasound transducer 3 may be held in a fixed spatial relationship with the cannula 2, such that the tip of the cannula 2 can be tracked very accurately.

The cannula insertion system 1 comprises a patient or treatment identification device 20. The patient or treatment identification device 20 is configured to read an identification of the patient. Such identification may for example be an identity card, token or chip, a passport, a hospital identity card, token or chip or a letter with barcode, or an insurance card. The patient or treatment identification device 20 may comprise any suitable reader or scanner to read/scan the information provided by the identification of the user. The patient or treatment identification device 20 may be connected with a central information system, such as an electronic patient file, a laboratory information system (LIS) or other system and may receive all data required for that patient for carrying out a cannula insertion procedure. After the identity of a patient has been determined, the identity of the patient may be confirmed, for example by providing an audible or readable output to the patient that has to confirmed by the patient.

The cannula insertion system 1 may comprise an emergency button that can be operated by the operator and/or the patient. When this emergency button is operated, the cannula insertion system will start abort procedure, in which the cannula insertion process is safely stopped.

The cannula insertion system 1 may further comprise a patient interface, for example a display device that can be seen by the patient.

The patient interface can be used to provide instructions to the patient, for example to reposition an arm, to provide information with respect to the cannula insertion procedure, such as the remaining time of the procedure, and/or display media, such as a video, that may distract the patient from the procedure. The patient interface may also be used for patient input. For example, the patient may be requested to respond to questions, such as the confirmation of the identity of the patient, the use of anticoagulation, etc.

Similarly, the cannula insertion system 1 may comprise an operator interface, such as a display device or communication device to provide relevant information with respect to the cannula insertion procedure to the operator. This operator interface may for example provide a sound or visual alarm system to inform the operator that the patient request help. This operator interface may comprise a digital communication device that sends data to a remote device, e.g. a smart watch or tablet device.

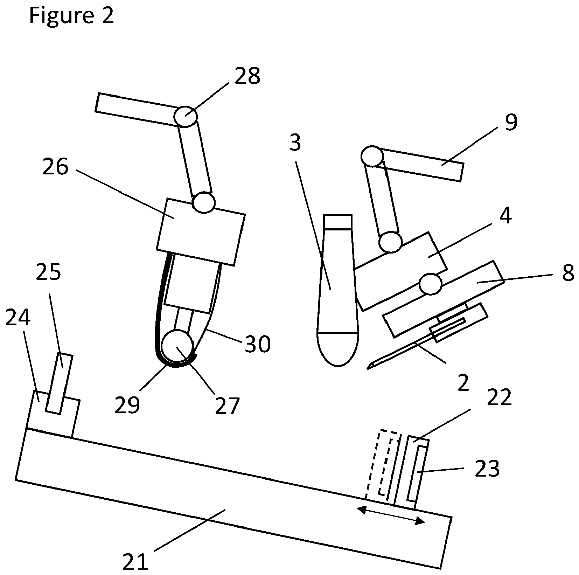

Figure 2 shows a second part of the cannula insertion system 1. The cannula insertion system 1 comprises an arm rest 21 on which the patient can place his arm. The armrest 21 may be adjustable in height based on the length/size of the patient. This height adjustment may be automatically or manually.

A hand grip 22 to be held by a hand of a patient is arranged on the armrest 21. The hand grip 22 is configured to measure a holding force with which the hand grip 22 is held by the patient. The hand grip 22 comprises a holding force sensor 23 with which this holding force can be measured. This holding force can be used to monitor the patient's well-being during the insertion of a cannula into the patient. It may be possible that a patient faints during the cannula insertion procedure.

To monitor the patient's well-being, the patient is requested to hold the hand grip 22 with a minimum holding force. This holding force is measured by the holding sensor 23. As long as the holding force is within a predefined holding force range, the procedure may be continued. As soon as the holding force is outside the predefined holding force range, i.e. below a lower threshold holding force or above an upper holding force threshold, the cannula insertion procedure may be aborted. In an alternative embodiment, one or more warning signals may first be provided to the patient to increase or decrease the holding force. When the holding force within a certain predefined time interval is not increased/decreased by the patient to a holding force within the predefined holding force range, the cannula insertion procedure may be aborted. In an alternative embodiment, the cannula insertion system may only provide warning signals when the holding force is outside the predefined holding force range.

In addition, or as an alternative, the hand grip 22 can be movable, for example slidable, between a standby-position (shown in solid lines) and an activated position (shown in dashed lines). The hand grip 22 may be biased, for example by a biasing spring, to the stand-by position. At the start of the procedure the patient is requested to move the hand grip 22 to the activated position. In this position, the cannula insertion system 1 may carry out a cannula insertion procedure. When the hand grip 22 is moved back into the stand-by position, for example when the patient faints and the biasing spring pushes the hand grip 22 back to the stand-by position, the cannula insertion system 1 is arranged to abort the cannula insertion procedure. Instead of the whole hand grip 22, it is also possible that only a part of the hand grip 22 is movable between a stand-by position and an activated position.

In an embodiment, the hand grip 22 could also comprise a humidity sensor arranged to measure humidity between the hand grip 22 and a hand of a patient in order to measure sweating of a patient. When increased sweating is determined by this humidity sensor a warning signal could be provided to the operator to check the patient.

Also, a camera can be provided in the cannula insertion system 1 to monitor the patient, for example to detect his face with for example face recognition. On the basis of face recognition, it may for example be determined that the patient faints. As soon as this is determined, a warning signal may be provided and/or the cannula insertion procedure may be aborted. Further, the cannula insertion system 1 may comprise one or more cameras or other sensors arranged to detect an object intruding into the workspace of the positioning system, for example a hand of a child and the cannula insertion system 1 may be arranged to abort the cannula insertion procedure when such intrusion is detected. Cameras or other sensors may also be arranged to detect the position of the arm of a patient. If the position of the arm of the patient is not correct, i.e. needs to be repositioned, a corresponding instruction can be provided to the patient or operator.

The cannula insertion system 1 further comprises a tourniquet device 24 to hold the upper arm of a patient during the cannula insertion procedure. The tourniquet device comprises a strap or band 25 which may be manually or automatically tightened on the upper arm of a patient. The pressure with which the tourniquet device 24 is automatically tightened around the upper arm of the patient may be made dependent on the patient itself, on the basis of the identification of the patient by the patient or treatment identification device 20 shown in Figure 1.

The tourniquet device 24 may also comprise an inflatable cuff that is tightened around the upper arm of a patient. In such embodiment, the inflatable cuff may also be used to measure a blood pressure of the patient.

The tourniquet device 24 may comprises a safety release device to release the tourniquet from a patient's arm or to release the tourniquet from the cannula insertion system if a pulling force on the tourniquet device 24 exceeds a pulling force threshold.

The safety release device could further be configured to release the upper arm when the time period in which the tourniquet device 24 is tightened around the upper arm of the patient exceeds a predefined maximum time period and/or when the pressure which is exerted by the tourniquet device 24 exceeds a predefined maximum pressure. This predefined maximum time period and/or predefined maximum pressure may be patient specific.

The safety release device may be configured to reduce the pressure exerted on the upper arm by loosening the strap or band 25, or by releasing the pressure in the inflatable cuff. Any other way or device to reduce the pressure within a short time period may also be applied.

The tourniquet device 24, or at least its band or strap 25 may be detachable so that it can be replaced and potentially be cleaned separately from the cannula insertion system 1. In an alternative embodiment, the strap or band 25 may be provided in a roll. The roll may be used to use for each patient a new strap or band 25 which is rolled from the roll. The tourniquet device 24 may also have a cleaning device to clean the tourniquet device 24 between two patients.

After retraction of the cannula 2 from the human or animal body, it may be needed to apply pressure on the penetration location where the cannula 2 has penetrated the skin to stop bleeding caused by the introduction of the cannula into the human or animal body. The cannula insertion system 1 comprises a pressure applicator 26 arranged to apply pressure on the penetration location of the cannula 2 after retraction of the cannula 2 from the insertion location. The pressure applicator 26 comprise a pressure element 27 which has a cylindrical shape wherein the longitudinal axis of the cylindrical shape is arranged substantially parallel to the skin surface on which a pressure should be exerted. In other embodiments the pressure element may be plate shaped or have any other suitable shape. The pressure applicator 26 is advantageously configured to automatically place, using the positioning system 28 the pressure element 27 at the penetration location and exert a suitable force with this pressure element 27.

In an alternative embodiment the pressure applicator 26 may be mounted on the end-effector 4 together with the cannula insertion device 8 and/or the ultrasound transducer 3.

The pressure applicator 26 comprises a bandage support to support a bandage 29 with which pressure is exerted on the insertion location. The pressure element 27 will press the bandage 29 against the skin at the penetration location to stop bleeding of the patient.

The use of a bandage 29 has the advantage that the pressure element may not have to be cleaned each time when the pressure element is used to exert a pressure on the skin. Since the bandage 29 will act as a barrier between the pressure element 27 and the insertion location in the arm of patient.

Furthermore, the bandage support, in this embodiment in the form of a carrier film 30 may be configured to hold the bandage 29 until the pressure applicator 26 is used to apply the pressure on the skin and then release the bandage 29. The bandage 29 may remain on the skin of the human or animal body to be used to further stop bleeding.

In an embodiment, the bandage may be a self-sealing bandage that is arranged on the skin surface before the insertion of the cannula into the patient. The cannula may be introduced into the patient through the bandage. After removal of the cannula, the bandage will seal the opening made by the cannula and seal the insertion location.

The carrier film 30 with releasable bandage 29 is rolled from a roll held by the pressure applicator 26. The carrier film 30 will be rolled up on another roll supported by the pressure applicator 26 after the bandage 29 is released from the carrier film 30. The bandage 29 may be made of any suitable material such as cotton of other known bandage material.

In an alternative embodiment, the roll with releasable bandage may be provided at any other suitable location of the cannula insertion system 1.

The pressure applicator 26 may be configured to apply the pressure during a predetermined time period. This may be a fixed time period, or a time period dependent on factors such as patient characteristics, such as age and anticoagulation factors, and other factors such as needle diameter. These factors may be known from the input at the patient or treatment identification device 20, or may be based on manual input from the patient or the operator.

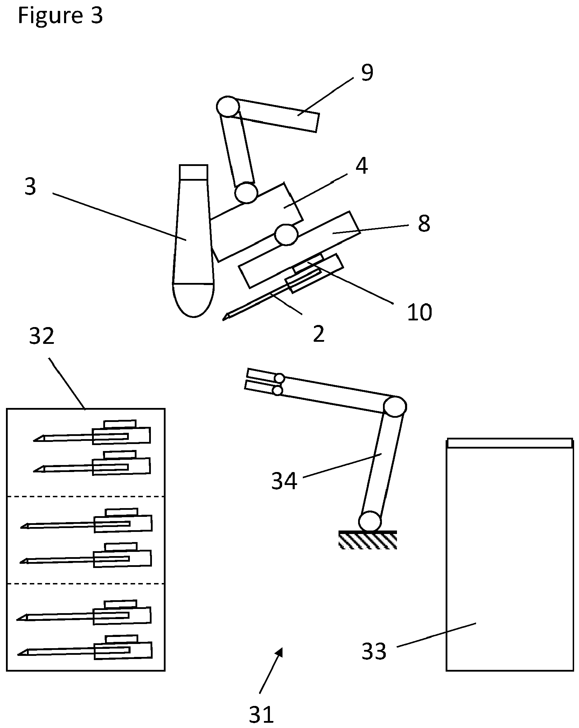

Figure 3 shows a part of the cannula insertion system 1 comprising a cannula exchange system 31 to exchange a cannula 2 held by the cannula insertion device 8. For each insertion of a cannula 2 into a human or animal body a new disposable cannula 2 should be used. The cannula exchange system 31 comprises a cannula supply 32 in which multiple cannulas 2 are stored and a cannula disposal container 33 in which used cannulas 2 may be disposed.

The cannula exchange system 31 comprises a displacement device 34, for example a robotic arm, to take a cannula 2 from a supply area at or near the cannula supply 32 where a cannula 2 is taken from the cannula supply 32 to a loading area where the cannula is loaded on the cannula insertion device 8 by coupling the cannula 2 to the cannula holder 10. After the cannula 2 is used the displacement device may take the cannula 2 from the cannula holder 10 to a discharge area where the cannula 2 may be discharged into the cannula disposal container 33.

The displacement device 34 is a separate movement device. In practice, the displacement device may have two robotic arms or other manipulators, one for taking a cannula 2 from the cannula supply 32 and coupling the cannula 2 on the cannula holder 10, and one for discharging the cannula 2 from the cannula holder into the cannula disposal container 33. One of the robotic arms or manipulators may be formed by the cannula insertion device positioning system.

In another embodiment, the positioning system 9 is used to move the cannula insertion device 8 to a suitable location to take a cannula 2 from a supply area at or near the cannula supply 32 and to discharge a used cannula 2 into the cannula disposal container 33. In such embodiment, the displacement 34 may be omitted.

The cannula disposal container may be any container suitable to receive cannulas after their use. The cannula disposal container may be disposable itself, so that it can be disposed together with the used cannulas, or the cannula can be emptied and placed back into the cannula insertion system, preferably after cleaning and disinfection.

The cannula supply 32 may comprise multiple types of cannulas 2 as shown in Figure 3, such as cannulas having different needle diameters or needle lengths. The cannula exchange system 31 is configured to select a cannula of a desired type. This selection may for example depend on the basis of an identity of a person in which the cannula 2 will be inserted, and an associated type of cannula 2. The selection of the cannula 2 may also depend on the size and location of the vein in which the cannula 2 will be inserted.

The cannula supply 32 may also comprise cannulas 2 that comprise at or near its distal end a blood contact sensor. Such blood contact sensor can be used to determine the actual entrance of the cannula into a blood vessel. By determining the actual entrance of the cannula into the blood vessel, this actual entrance can be compared with the expected entrance of a blood vessel on the basis of the cannula insertion path as determined by the control device of the cannula insertion system.

This confirmation of the entrance of the blood vessel can be used to determine whether the cannula insertion path is correctly followed. When there is a large difference between the actual entrance of a blood vessel as measured with a blood contact sensor and the expected entrance of a blood vessel on the basis of the cannula insertion path, the cannula insertion procedure may be stopped as a safety measure.

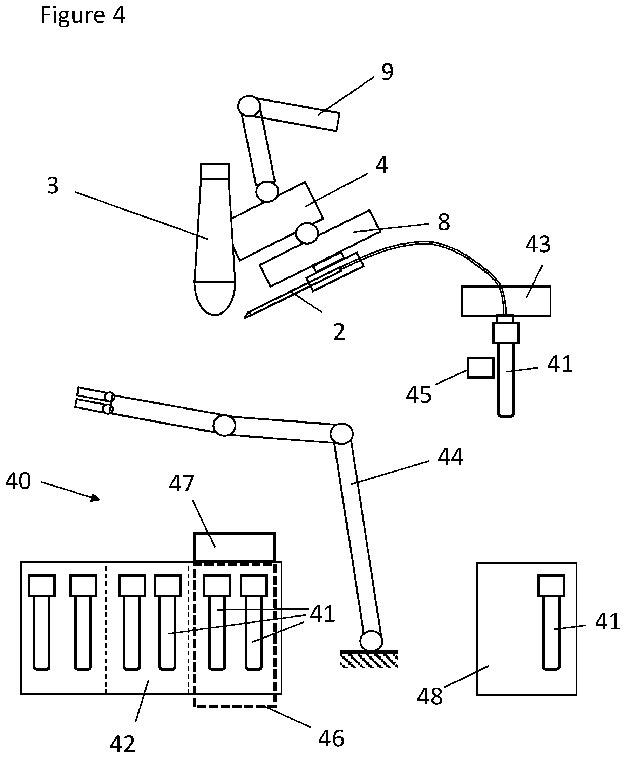

Figure 4 shows a blood collection tube exchange system 40 that can be used in the cannula insertion system 1 to exchange blood collection tubes 41.

During blood drawing of blood from a patient a blood collection tube 41 is connected to the cannula 2. The blood collection tube 41 is for example held by a tube holder 43 of a blood collection system.

A blood quantity sensor 45 may be arranged to provide a blood quantity signal representative for a quantity of blood present in the blood collection tube 41. With the blood quantity sensor 45 it can be determined whether the blood collection tube 41 is filled with blood to a desired filling level. The blood quantity sensor 45 may be an optical sensor that can determined a blood level in the blood collection tube 41. Other types of sensors may also be used to determine the quantity of blood in the blood collection tube 41.

During blood withdrawal, it may be desirable that multiple blood collection tubes are filled with blood.

The blood collection tube exchange system 40 is configured to select the respective blood collection tubes from a blood collection tube storage 42 in which multiple types of blood collection tubes 41 may be stored. The blood collection tube storage 42 may also function as a blood collection tube input device into which blood collection tubes are manually introduced.

It may be desirable that, for a specific patient, blood collections tubes of a set of pre-selected blood collections tubes 41 are subsequently filled with blood drawn from a blood vessel of one patient. The set of pre-selected blood collections tubes 41 may be arranged in a cartridge 46. The cartridge 46 may define one or more holding positions for holding the one or more pre-selected blood collection tubes 41. The cartridge 46 may be designed in such a way that the one or more blood collection tubes can manually be easily arranged in the cartridge 46, but that it relatively hard to take the blood collection tubes manually out of the cartridge 46 . This may prevent that a person may advertently or inadvertently one or more blood collection tubes 41 out of the cartridge 46. A blood collection tube removal device 47 may be provided in the cannula insertion system 1 to remove the blood collection tube 41 out of the cartridge once place in the cannula insertion system 1. The blood collection tube removal device 47 may comprise one or more pushers that are capable of pushing a blood collection tube 41 out of the cartridge 46.

In an embodiment, the blood collection tube exchange system 40 may further be configured to couple a selected blood collection tube 41 to the cannula to fill the blood collection tube to a desired filling level.

When the blood collection tube 41 is sufficiently filled, the blood collection tube 41 may be transferred to a blood collection tube release device 48, where the blood collection tube 41 can be taken out of the cannula insertion system 1.

The blood collection tube exchange system comprises a manipulator 44 to take a blood collection tube 41 out of the blood collection tube storage 42. In this embodiment, the manipulator 44 is arranged to couple the selected blood collection tube 41 to the cannula 2 by placing it in the tube holder 43. After the blood collection tube 41, is filled with blood to the desired level, the manipulator 44 may take the blood collection tube 41 from the tube holder 43 and transport it to blood collection tube release device 48. In practice, the blood collection tube storage 42 and the blood collection tube release device 48 may be integrated in a single device.

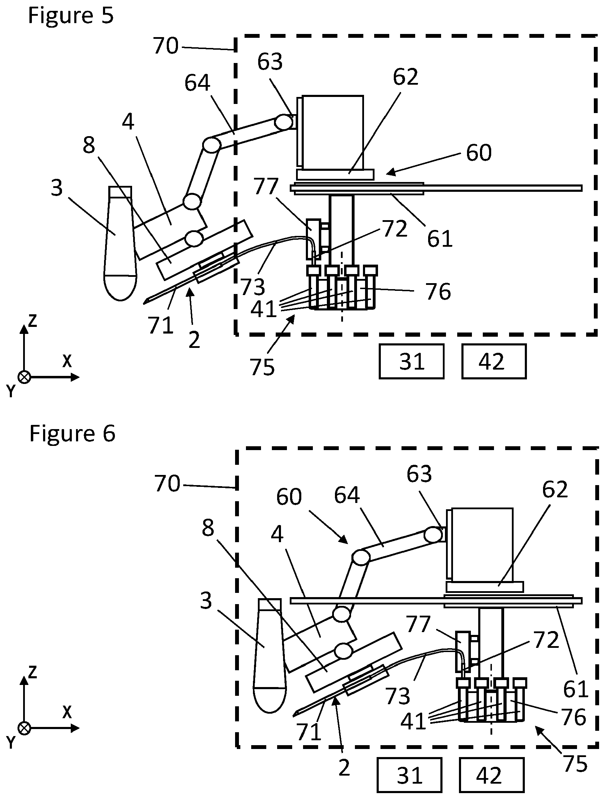

In another embodiment, the blood collection tube exchange system 40 and its manipulator 44 are used to load and unload the blood collection tubes 41 in and out a blood collection system as described with respect to the embodiment of Figures 5-7. In this embodiment, another device may be used to connect the cannula 2 to the respective blood collection tube 41.

The tube exchange system 40 may also be configured to invert, when desired, the filled blood collection tube 41 in order to mix a coagulant present in the blood collection tube 41 with the blood.

In an alternative embodiment, the positioning system 9 may be used to manipulate the blood collection tubes 41, for example to take a blood collection tube 41 out of the blood collection tube storage 42 and couple the selected blood collection tube 41 to the cannula 2 and/or, after filling, to take the blood collection tube 41 from the tube holder 43 and transport it to blood collection tube release device 48. In such embodiment, the manipulator 44 may be omitted.

The blood collection tube release device 48 may be arranged to label the blood collection tube 41. The blood collection tube release device 48 may also be configured to mix the content of blood tubes in a standardized way and/or to centrifuge the blood tube to separate serum and plasma from blood after blood draw so the time period until blood analysis is performed can be extended. The blood collection tube release device 48 may also be configured to resolve any remaining pressure difference of the filled blood collection tubes with the environment, in order to maintain the blood quality and prevent hemolysis.

The blood collection tube release device 48 may further be configured to perform blood test analysis after the blood is drawn at point-of-care. For example, a HIL test on Hemolysis, Icterus and Lipemia can be performed to determine sample quality.

In an embodiment, the blood collection tube release device 48 can be connected to a tube system (blood tube transportation system, e.g. pneumatic) that is directly connected with a laboratory.

In an embodiment, the filled blood collection tubes 41 can be placed back into the cartridge 46.

For hygienic reasons, the end-effector 4 and/or the ultrasound transducer 3 may be placed in a cover to protect against blood and bodily fluids. The cover could be a plastic cover, a bandage or a special fabricated shield. The cannula insertion system 1 may be arranged to automatically arrange the cover on the end-effector 4 and/or the ultrasound transducer 3 and to dispose it when desired.

Sensors, e.g. the cameras are able to check if the procedure is according plan and they can check if there are no blood drips on the armrest before the procedure starts/ after the procedure.

In case of blood drips on arm rest, the device can signal to the operator that cleaning and disinfection is required.

The device can clean and disinfect itself, in particular the parts touching the patient at the venipuncture location, especially in the case of contact sensors

This cleaning can be done by automated brushing, washing, or wiping of (part of) the end-effector.

After cleaning, the parts can be dried to prepare for disinfection, for example through blowing of air or heating.
Disinfection may occur through closed UV-C chamber for a predetermined amount of time; alternatively, disinfection occurs through automated spraying, wiping and/or soaking.

Figures 5 and 6 show an alternative embodiment of a positioning system 60 for positioning of the end-effector 4. The positioning system 60 comprises a first stage 61 to move the end-effector 4 in x-direction, a second stage 62 to move the end-effector 4 in y-direction and a third stage 63 to move the end-effector in z-direction, wherein the x-direction, y-direction and z-direction are orthogonal directions. The positioning system 60 further comprising a connection arm 64 comprising three rotation actuators to rotate the end-effector 4 about each of the x-direction, y-direction and z-direction. This results in end-effector 4 being movable in six degrees of freedom to optimally position the end-effector 4 with respect to a human or animal body, for example a patient's arm.

The first stage 61 has a relatively long stroke that can be used to move the end-effector 4 between an operating position (Figure 5) from which a cannula 2 may be inserted into the human or animal body, and a retracted position (Figure 6). In dashed lines a housing 70 of the cannula insertion system 1 is shown. This housing 70 comprises a recess in which the cannula insertion device may at least partially be placed. During the insertion of the cannula 2 into a patient, the first stage 61 may be arranged in the operating position in which the end-effector 4 extends at least partially outside the housing 70. After blood withdrawal is finished, the end-effector 4 including the cannula insertion device 8 may be moved to the retracted position so that the cannula insertion device 8 no longer extends from the housing 70. In the retracted position, within the housing 70, the cannula insertion system 1 may exchange disposable parts such as a cannula and blood collection tubes filled during blood withdrawal. Also, new disposable parts may be provided to facilitate blood withdrawal of a subsequent patient. For example, in the retracted position of the end-effector 4, the cannula 2 and the blood collection tubes 41 may be exchanged.

The cannula 2 comprises a patient needle 71 to be inserted into a patient, a tube needle 72 to be inserted in a blood collection tube 41 and a conduit 73 connecting the patient needle 71 and the tube needle 72. The cannula 2 is provided as a disposable part. To fill a blood collection tube 41 with blood the tube needle 72 is inserted into the blood collection tube 41 and the patient needle 71 into a blood vessel of the human or animal body. Advantageously, the conduit 73 between the patient needle 71 and the tube needle 72 is relatively short so that a relatively low volume of blood is needed to fill the conduit 73. For this reason, the blood collection tubes 41 are held by a blood collection system 75 which is mounted on the first stage 61 of the positioning system 60. As a result, the blood collection system 75 will move together with the end-effector 4 between the operating position and the retracted position and will stay relatively close to the end-effector 4.

The blood collection system 75 comprises a carousel 76 comprising multiple holding locations each arranged to hold a blood collection tube 41 and a tube needle insertion device 77 to insert the tube needle 72 in a blood collection tube 41 aligned with the and pull the tube needle out of the blood collection tube 41 after the blood collection tube 41 has been filled with blood. The carousel 76 is rotatable about an axis of rotation extending in z-direction. By rotation about the axis of rotation a selected one of the holding locations can be aligned with the tube needle insertion device 77 to allow the tube needle 72 to be inserted into the blood collection tube held in the selected one of the holding locations. When the blood collection tube 41 is filled, the tube needle insertion device 77 may retract the tube needle 72 out of the blood collection tube 41 and a new holding location and blood collection tube 41 held therein may be aligned with the tube needle insertion device 76 by rotation of the carousel 76. When arranged in this position the tube needle insertion device 76 may insert the tube needle 72 into this new blood collection tube 41. To determine whether a blood collection tube is sufficiently filled a sensor may be provided that is capable of providing a level signal representative for sufficient filling of the blood collection tube 41. This sensor is for example a level sensor that can measure whether a predetermined level of blood in the blood collection tube 41 has been reached, for example by using a light beam.

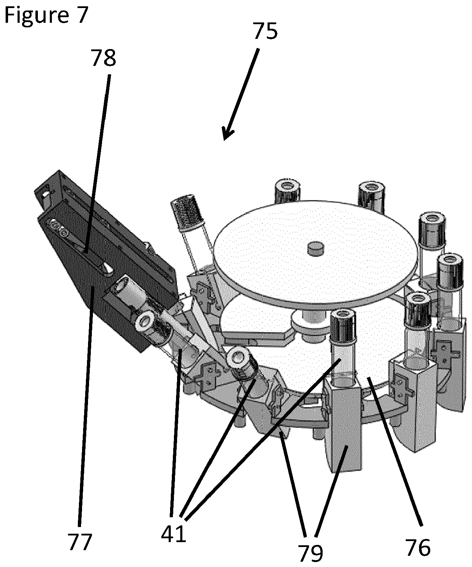

Figure 7 shows an actual embodiment of a blood collection system 75 that can be mounted on the first stage 61. The blood collection system 75 comprises a carousel 76 and a needle insertion device 77. The carousel 76 comprises multiple holding locations each arranged to hold a blood collection tube. The tube needle insertion device 77 comprises an actuator 78 to insert and withdraw the tube needle 72 into and from the blood collection tube 41 aligned with the needle insertion device 77. The tube holding locations are formed by tube holders 79 that are mounted tiltable on the carousel 76. To properly align the blood collection tubes 41 with the tube needle insertion device 77 of this embodiment, the tube holders 79 and the blood collection tubes 41 held therein are tilted with their top ends radially outwardly with respect to the axis of rotation of the carousel 76.

In an embodiment, the blood collection system 75 may comprise an actuator to invert and/or rotate a blood collection tube during or after being filled with blood from the human or animal body.

Again referring to Figures 5 and 6, in the retracted position of the end-effector 4, the cannula exchange system 31 is provided to exchange a cannula 2 held by the cannula insertion device 8 and a blood collection tube exchange system 40 to exchange blood collection tubes 41 held by the blood collection system 75. The cannula exchange system 31 may for example be embodied as described with respect to Figure 3. The blood collection tube exchange system 40 may for example be embodied as described with respect to Figure 4.

Embodiments of different aspects of the invention can be described as follows:
1. A cannula insertion system to insert a cannula into a human or animal body, comprising:
   one or more sensors to determine a suitable location for insertion of the cannula,
   a cannula insertion device configured to insert the cannula into the human or animal body,
   a cannula insertion device positioning system to support and position the cannula insertion device, and
   a control device arranged to control the positioning system to position, on the basis of the determined location for insertion of the cannula, the cannula insertion device in a suitable position to insert the cannula into human or animal body.
2. The cannula insertion system of embodiment 1, wherein the cannula insertion system comprises a blood collection system to collect blood in one or more blood collection tubes supported by the blood collection system,
   wherein the blood collection system is movably arranged to at least partly follow movements of the cannula insertion device,
   wherein the blood collection system for example is mounted on the cannula insertion device positioning system to at least partly follow movements of the cannula insertion device.
3. The cannula insertion system of embodiment 1 or 2, wherein the cannula insertion device positioning system is arranged to move the cannula insertion device between an operating position from which a cannula may be inserted into the human or animal body, and a retracted position.
4. The cannula insertion system of any of the preceding embodiments, wherein the cannula insertion device positioning system comprises at least one movable stage, wherein the blood collection system is mounted on the at least one stage.
5. The cannula insertion system of any of the preceding embodiments, wherein the cannula insertion system comprises a blood collection tube exchange system to exchange blood collection tubes between a blood collection tube input device and the blood collection system.
6. The cannula insertion system of any of the embodiment 2 - 5, wherein the blood collection system comprises an actuator to invert and/or rotate a blood collection tube during or after being filled with blood from the human or animal body.
7. The cannula insertion system of any of the preceding embodiments, wherein each cannula comprises a patient needle to be inserted into a patient, a tube needle to be inserted in a blood collection tube and a conduit connecting the patient needle and the tube needle.
8. The cannula insertion system of embodiment 3 and 5, wherein, in the retracted position, the blood collection tube exchange system may exchange one or more blood collection tubes supported by the blood collection system.
9. The cannula insertion system of any of the preceding embodiments, wherein the cannula insertion system comprises a cannula exchange system to exchange a cannula held by the cannula insertion device, and wherein the cannula exchange system is arranged to dispose a used cannula in a cannula disposal container.
10. The cannula insertion system of embodiment 3 and 9, wherein, in the retracted position, the cannula exchange system may exchange a cannula with the cannula insertion device.
11. The cannula insertion system of any of the embodiments 9-10, wherein the cannula exchange system is arranged to select the cannula from a cannula supply comprising multiple cannulas.
12. The cannula insertion system of any of the preceding embodiments, wherein the suitable location is located on or in an arm of a human, and wherein the one or more sensors are arranged to determine a shape of the arm and/or a location of an elbow pit of the human.
13. The cannula insertion system of any of the preceding embodiments, wherein the cannula insertion system comprises an end-effector supporting the one or more sensors and the cannula insertion device.
14. The cannula insertion system of any of the preceding embodiments, wherein the cannula insertion system is arranged to insert a cannula into a vein of the human or animal body.
15. The cannula insertion system of the preceding embodiment, wherein the one or more sensors provide at least one sensor signal, and wherein the control device is arranged to create on the basis of the at least one sensor signal an image of the vein suitable for insertion of the cannula.
16. The cannula insertion system of any of the preceding embodiments, wherein the cannula insertion system comprises a pressure applicator arranged to apply pressure on the insertion location after removal of the cannula from the insertion location.
17. The cannula insertion system of the preceding embodiment, wherein the pressure applicator comprises a bandage support to support a bandage with which pressure is exerted on the insertion location.
18. The cannula insertion system of any of the preceding embodiments, wherein the system comprises a patient or treatment identification device.
19. The cannula insertion system of any of the preceding embodiments, wherein the cannula insertion system comprises a hand grip to be held by a hand of a patient, wherein the hand grip is configured to measure a holding force with which the hand grip is held by the patient and/or a holding position in which the hand grip is held by the patient.
20. The cannula insertion system of the preceding embodiment, wherein the cannula insertion system is arranged to carry out a cannula insertion procedure if the holding force is within a predefined holding force range, and to abort the cannula insertion procedure if the holding force is outside the predefined holding force range.
21. The cannula insertion system of embodiment 19 or 20, wherein the hand grip, or at least a part thereof, is movable between a stand-by position and an activated position, wherein the cannula insertion system is arranged to carry out a cannula insertion procedure if the hand grip or the movable part thereof is arranged in the activated position, and to abort the cannula insertion procedure if the hand grip or the movable part thereof is arranged in the stand-by position.
22. The cannula insertion system of any of the preceding embodiments, wherein the cannula insertion system comprises a tourniquet device, wherein the tourniquet comprises a safety release device to release the tourniquet from a patient's arm or to release the tourniquet from the cannula insertion system if a pulling force exceeds a pulling force threshold.
23. The cannula insertion system of any of the preceding embodiments, wherein the blood collection tube exchange system comprises a blood quantity sensor arranged to provide a blood quantity signal representative for a quantity of blood present in a blood collection tube.
24. The cannula insertion system of any of the preceding embodiments, wherein the one or more sensors are arranged to determine one or more locations of nerves, and wherein the control device is arranged to prevent the insertion of the cannula into the nerves.
25. The cannula insertion system of any of the preceding embodiments, wherein the cannula insertion system comprises a blood presence sensor located near, on or in the cannula arranged to determine the presence of blood in the cannula.

## Claims

1. A cannula insertion system to insert a cannula into a human or animal body, comprising:
one or more sensors to determine a suitable location for insertion of the cannula,
a cannula insertion device configured to insert the cannula into the human or animal body,
a cannula insertion device positioning system to support and position the cannula insertion device, and
a control device arranged to control the positioning system to position, on the basis of the determined location for insertion of the cannula, the cannula insertion device in a suitable position to insert the cannula into human or animal body.

2. The cannula insertion system of claim 1, wherein the cannula insertion device comprises a blood collection tube exchange system configured to couple a selected blood collection tube to the cannula or to exchange blood collection tubes between a blood collection tube input device and a blood collection system.

3. The cannula insertion system of claim 1, wherein the cannula insertion system comprises a blood collection system, wherein the blood collection system comprises an actuator to invert and/or rotate a blood collection tube during or after being filled with blood from the human or animal body.

4. The cannula insertion system of claim 3, wherein the cannula insertion device comprises a blood collection tube exchange system configured to exchange blood collection tubes between a blood collection tube input device and the blood collection system

5. The cannula insertion system of claim 2, wherein the blood collection tube exchange system is configured to invert, when desired, a filled blood collection tube.

6. The cannula insertion system of any of the claims 1-5, wherein the cannula insertion system comprises a blood collection tube input device to introduce blood collection tubes into the cannula insertion system.

7. The cannula insertion system of claim 6, wherein the blood collection tube input device is configured to receive a cartridge comprising a set of pre-selected blood collections tubes.

8. The cannula insertion system of claim 7, wherein the cartridge defines one or more holding positions for holding the one or more pre-selected blood collection tubes of the set of pre-selected blood collections tubes.

9. The cannula insertion system of claim 7 or 8, wherein the cartridge is designed in such a way that the one or more blood collection tubes can be taken out of the cartridge using a blood collection tube removal device or a cartridge opening device.

10. The cannula insertion system of any of the claims 1-9, wherein the cannula insertion system comprises a pressure applicator arranged to apply pressure on the insertion location after retraction of the cannula from the insertion location.

11. The cannula insertion system of claim 10, wherein the pressure applicator comprises a bandage support to support a bandage with which pressure is exerted on the insertion location.

12. The cannula insertion system of any of the preceding claims, wherein the blood collection tube exchange system or blood collection system comprises a blood quantity sensor arranged to provide a blood quantity signal representative for a quantity of blood present in a blood collection tube.

13. The cannula insertion system of any of the preceding claims, wherein the cannula insertion system is arranged to verify whether a detected blood vessel is a vein and not an artery.

14. A cartridge for use in a cannula insertion system to insert a cannula into a human or animal body, wherein the cartridge is configured to hold a set of pre-selected blood collections tubes.

15. The cartridge of claim 14, wherein the cartridge is designed in such a way that the one or more blood collection tubes can be taken out of the cartridge using a blood collection tube removal device or a cartridge opening device.
